(19) Europäisches Patentamt
European Patent Office
Office européen des brevets

(11) **EP 2 684 515 B1**

(12) **FASCICULE DE BREVET EUROPEEN**

(45) Date de publication et mention
de la délivrance du brevet:
**17.12.2014 Bulletin 2014/51**

(51) Int Cl.:
***A61B 5/02*** *(2006.01)* ***A61B 5/024*** *(2006.01)*

(21) Numéro de dépôt: **13172888.3**

(22) Date de dépôt: **19.06.2013**

(54) **Dispositif médical actif comprenant des moyens de suivi de l'état d'un patient souffrant d'un risque d'insuffisance cardiaque**

Aktive medizinische Vorrichtung, die Mittel zur Überwachung des Zustands eines Patienten mit Herzinsuffizienzrisiko umfasst

Active medical device comprising means for monitoring the condition of a patient suffering from a risk of heart failure

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.07.2012 FR 1256792**

(43) Date de publication de la demande:
**15.01.2014 Bulletin 2014/03**

(73) Titulaire: **Sorin CRM SAS**
**92140 Clamart (FR)**

(72) Inventeurs:
• **Baumann, Oliver**
**Southampton, SO19 4BZ (GB)**
• **Giorgis, Lionel M.**
**22000 SAINT BRIEUC (FR)**

(74) Mandataire: **Dupuis-Latour, Dominique**
**Bardehle Pagenberg**
**10, boulevard Haussmann**
**75009 Paris (FR)**

(56) Documents cités:
**EP-A1- 0 347 708       EP-A1- 2 092 885**
**EP-A2- 1 867 360       WO-A1-2009/107007**
**US-A1- 2007 043 299    US-A1- 2007 239 218**
**US-A1- 2010 023 081    US-B1- 7 848 793**

**Description**

L'invention concerne les "dispositifs médicaux actifs" tels que définis par la directive 93/42/CE du 14 juin 1993 du Conseil des communautés européennes.

Cette définition inclut les dispositifs implantables de stimulation cardiaque, resynchronisation et/ou défibrillation destinés au diagnostic et au traitement des troubles du rythme cardiaque.

Elle inclut également les dispositifs actifs, implantés ou non, à visée purement diagnostique, notamment les systèmes externes de suivi à domicile (*home monitoring*) des patients, mettant en oeuvre un appareil d'interrogation à distance qui est activé à intervalles périodiques, par exemple quotidiennement, pour télécharger les données recueillies par un implant et les transmettre pour analyse à un site distant de télésurveillance. L'invention concerne plus particulièrement le suivi des patients atteints d'insuffisance cardiaque, ou en risque d'insuffisance cardiaque. L'insuffisance cardiaque est une pathologie complexe, avec des causes et des effets divers, incluant notamment régurgitation de la valvule mitrale, cardiomyopathie dilatée et ischémie.

Il s'agit de phénomènes évolutifs et délétères qu'il est important de pouvoir évaluer et suivre au cours du temps, en particulier pour émettre des alertes avancées en cas de tendance à l'aggravation.

L'évolution peut notamment résulter d'un phénomène dénommé "remodelage cardiaque", qui peut être défini comme l'ensemble des modifications du coeur en réponse à une pathologie, et qui est généralement associé à un plus mauvais pronostic. Ce remodelage se manifeste à la longue par une augmentation de la taille du ventricule gauche, avec dégradation de la fraction d'éjection du fait de la baisse de contractilité, et *in fine* une diminution du débit cardiaque entrainant de graves conséquences sur l'organisme par progression de l'insuffisance cardiaque.

Outre les alertes en cas d'aggravation de l'état du patient, il peut également s'agir de modifier le paramétrage d'un stimulateur, notamment dans le cas d'un dispositif assurant une stimulation conjointe et permanente des ventricules gauche et droit afin de resynchroniser ces derniers, technique dite CRT (*Cardiac Resynchronization Therapy*) ou BVP (*BiVentricular Pacing*). En effet, les effets bénéfiques procurés par la thérapie CRT peuvent conduire, à terme, à réévaluer la thérapie appliquée et modifier la configuration primitive et le paramétrage du resynchroniseur.

La technique de référence pour l'évaluation des pathologies indiquées plus haut (régurgitation valvulaire, dilatation du ventricule gauche, etc.) est l'évaluation par échocardiographie. Cette procédure doit être mise en oeuvre par un praticien qualifié et elle est de ce fait longue et couteuse, de sorte qu'elle ne peut pas être appliquée aussi souvent que cela serait utile ou nécessaire.

Diverses techniques de suivi et de diagnostic automatisées ont été proposées, notamment par le EP 1 867 360 A2 (ELA Medical), qui propose de recouper diverses informations issues de capteurs de ventilation-minute (capteur dit "MV"), d'activité (capteur accélérométrique "G") et d'accélération endocardiaque (EA) ou de bioimpédance intracardiaque. Des algorithmes d'analyse produisent des indicateurs de risque de décompensation cardiaque, et des moyens d'analyse croisée délivrent un signal composite d'alerte préventive, sur différents niveaux, en fonction des indicateurs produits par les algorithmes.

Le US 2007/043299 A1 décrit un dispositif mettant en oeuvre une technique comparable, basée sur l'analyse croisée de l'amplitude crête-à-crête du premier pic d'accélération endocardiaque et à la fréquence cardiaque, pour évaluer la progression de l'insuffisance cardiaque chez un patient appareillé du dispositif.

Ces algorithmes sont efficaces pour évaluer globalement la contractilité cardiaque du patient et la dégradation plus ou moins rapide de cette contractilité, correspondant à différents degrés d'alerte. En revanche, ils ne permettent pas d'évaluer de façon plus spécifique l'apparition ou l'évolution de certaines pathologies particulières telles que celles évoquées plus haut (ischémie, régurgitation de la valvule mitrale, etc.).

Il serait donc souhaitable de pouvoir disposer de moyens de suivi et de diagnostic qui puissent être plus particulièrement mis en oeuvre en fonction de la pathologie propre du patient, de manière à délivrer si besoin une alerte spécifique.

À cet effet, l'invention propose un dispositif du type général divulgué par le US 2007/043299 A1 précité, c'est-à-dire comportant : des moyens de mesure de la fréquence cardiaque ; des moyens de recueil d'un signal d'accélération endocardiaque ; des moyens aptes à dériver du signal d'accélération endocardiaque un paramètre hémodynamique représentatif de la contractilité du myocarde ; des moyens d'acquisition, aptes à définir au moins des couples de valeurs {fréquence, paramètre} au cours de cycles cardiaques successifs, ou de séries successives de cycles cardiaques ; des moyens classificateurs, aptes à distribuer lesdits couples de valeurs dans des classes de fréquence prédéterminées d'un profil discrétisé de valeurs {fréquence, paramètre} ; et des moyens d'analyse dudit profil, aptes à délivrer un indice (a, b) représentatif d'un état clinique du patient.

De façon caractéristique, le paramètre hémodynamique représentatif de la contractilité du myocarde est un paramètre temporel représentatif de l'intervalle de temps séparant le premier et le second pic d'accélération endocardiaque.

Le choix de ce paramètre est en effet particulièrement avantageux pour apprécier le degré d'ischémie du patient et l'évolution spécifique de cette pathologie, indépendamment de l'évolution générale de son insuffisance cardiaque. Des études cliniques ont en effet montré une modification notable de l'intervalle entre les deux pics lorsque survient un événement ischémique consécutif à l'occlusion d'une artère coronaire.

Dans une forme de mise en oeuvre, les moyens d'analyse du profil sont des moyens aptes à extraire au moins une donnée représentative du profil courant du patient, et à comparer cette donnée représentative courante à une donnée représentative de référence prédéterminée. La donnée représentative de référence peut être une donnée représentative obtenue à partir d'au moins un profil antérieur du patient, ou bien être issue de valeurs recueillies auprès d'une population connue de patients sains.

De préférence, les moyens d'analyse appliquent, avant comparaison à la donnée représentative de référence, une pondération prédéterminée à chaque classe de profil.

Ces moyens d'analyse peuvent notamment modéliser le profil courant par une régression linéaire ou quadratique, les paramètres de cette régression formant lesdites données de profil.

Dans une forme de mise en oeuvre plus élaborée, le dispositif comprend en outre des moyens discriminateurs des phases d'activité du patient, notamment trois phases d'activité du patient (effort, repos et récupération), et les moyens d'acquisition définissent des triplets de valeurs {fréquence, paramètre, activité} au cours des cycles cardiaques successifs, ou séries successives de cycles cardiaques. Le profil est un profil tridimensionnel discrétisé de valeurs {fréquence, paramètre, activité}, les moyens classificateurs plaçant les triplets de valeurs dans des classes de fréquence prédéterminées du profil tridimensionnel.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1 reproduit une série de relevés donnant l'amplitude du pic d'accélération endocardiaque en fonction de la fréquence cardiaque, avant et après une angioplastie, obtenus expérimentalement pour un patient atteint de régurgitation de la valvule mitrale.

La Figure 2 montre divers chronogrammes de signaux d'électrogramme EGM et d'accélération endocardiaque EA relevés expérimentalement sur un animal sain, avec occlusion progressive d'une artère coronaire.

La Figure 3 est une représentation schématique du dispositif de l'invention, illustrant l'enchaînement des différentes étapes de traitement des données recueillies.

La Figure 4 est un exemple de profil bidimensionnel de l'amplitude du pic du signal EA en fonction de la fréquence cardiaque, pour un patient sain et pour un patient souffrant de régurgitation de la valvule mitrale.

La Figure 5 illustre un exemple de profil bidimensionnel donnant l'intervalle de temps séparant le premier pic du deuxième pic du signal EA en fonction de la fréquence cardiaque, pour un patient sain et pour un patient ischémique.

La Figure 6 est un diagramme illustrant l'introduction d'un critère d'analyse supplémentaire effort/repos/récupération, avec les transitions entre les différents états.

La Figure 7 est un exemple de profil tridimensionnel donnant l'amplitude du pic du signal EA en fonction à la fois de la fréquence cardiaque et de l'état d'activité du patient.

On va maintenant décrire un exemple de réalisation de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply* et *Paradym* produits et commercialisés par Sorin CRM, Clamart, France, éventuellement associés à un dispositif externe de suivi à domicile (*home monitoring*) du patient tel que le *Smartview Remote Monitoring System,* également de Sorin CRM.

Il s'agit de dispositifs à microprocesseur programmable comportant des circuits pour recevoir, mettre en forme et traiter des signaux électriques recueillis par des électrodes implantées et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens principalement logiciels, au moyen d'algorithmes appropriés exécutés par un micro-contrôleur ou un processeur numérique de signal. Pour la clarté de l'exposé, les divers traitements appliqués seront décomposés et schématisés par un certain nombre de blocs fonctionnels distincts, mais cette représentation n'a toutefois qu'un caractère illustratif, les fonctions de recueil et de traitement des signaux pouvant être mises en oeuvre par un ou plusieurs logiciels au sein de l'implant et/ou du dispositif externe.

La technique de l'invention est basée sur l'analyse de l'accélération endocardiaque (ci-après "EA"), qui est un paramètre qui reflète très précisément et en temps réel les phénomènes concourant au fonctionnement mécanique du myocarde et qui peut être mesuré par un accéléromètre couplé au muscle cardiaque, comme cela est décrit par exemple dans le EP 0 515 319 A1 (Sorin Biomedica Cardio SpA). Ce document enseigne la manière de recueillir un signal EA au moyen d'une sonde endocavitaire pourvue d'une électrode distale de stimulation implantée dans l'oreillette ou le ventricule et intégrant un microaccéléromètre permettant de mesurer l'accélération endocardiaque.

On notera toutefois que, bien que dans la présente description on fasse principalement référence à l'analyse d'un signal EA délivré par un capteur placé sur une sonde endocavitaire, l'invention est également applicable à une analyse opérée à partir d'un signal EA délivré par d'autres types de capteurs implantés, tels qu'un capteur de mouvement d'une paroi du myocarde, un capteur épicardique ou un accéléromètre placé dans le boitier d'un implant. L'invention est également applicable à l'analyse d'un signal EA externe recueilli de manière non invasive, par exemple issu d'un capteur fixé sur la poitrine du patient au niveau du sternum.

Le signal EA recueilli au cours d'un cycle cardiaque donné forme deux composantes principales, correspondant aux deux bruits majeurs du coeur (sons "S1" et "S2" du phonocardiogramme) qu'il est possible de reconnaitre dans chaque cycle cardiaque :

- la composante EA1, débutant à la suite du complexe QRS, est engendrée par une combinaison de la fermeture des valves auriculo-ventriculaires, de l'ouverture des valves semi-lunaires et de la contraction du ventricule gauche. Les variations d'amplitude de cette composante EA1 sont étroitement liées aux variations de la pression dans le ventricule (l'amplitude maximale crête-à-crête étant plus précisément corrélée au maximum positif de la variation de pression dP/dt dans le ventricule gauche) et peuvent donc constituer un paramètre représentatif de la contractilité du myocarde, elle-même liée au niveau d'activité du système sympathique ; et
- la composante EA2 survient pendant la phase de relaxation ventriculaire isovolumique. Elle accompagne la fin de la systole ventriculaire et est principalement produite par la fermeture des valves aortiques et pulmonaires.

Il est possible d'extraire du signal EA recueilli un certain nombre de paramètres représentatifs, notamment :

- l'amplitude crête-à-crête de la composante EA1, désignée ci-après "PEA1 ", c'est-à-dire l'amplitude du premier pic d'accélération ; et
- l'intervalle de temps séparant la composante EA1 de la composante EA2, ci-après désignée "Ts". Cet indicateur, qui correspond à la durée de la systole, est généralement compté entre l'instant marquant le début de la composante EA1 et l'instant marquant le début de la composante EA2

Le EP 2 092 885 A1 (ELA Medical) décrit en détail des techniques d'analyse d'un signal EA permettant, entre autres, d'extraire ces deux informations PEA1 et Ts, et l'on pourra se référer à ce document pour plus de détails sur la manière dont sont déterminés ces paramètres, ainsi que d'autres caractéristiques des composantes EA1 et EA2.

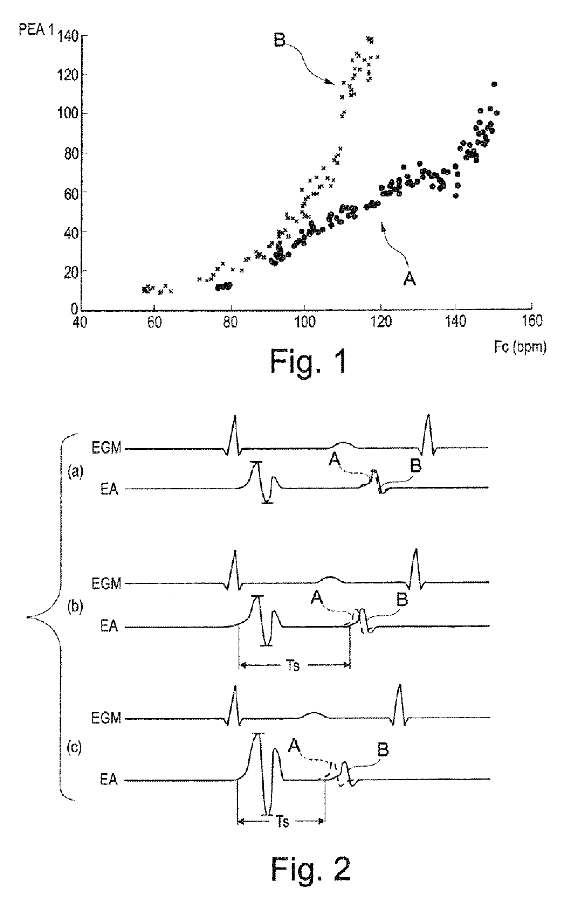

Sur la Figure 1, on a illustré une série de relevés donnant la valeur de PEA1 en fonction de la fréquence cardiaque Fc obtenus expérimentalement pour un patient atteint de régurgitation de la valvule mitrale, avant et après une angioplastie. Les points A correspondent aux relevés pour un patient présentant une régurgitation sévère de la valve mitrale, et les croix B sont ceux obtenus pour ce même patient, après une intervention d'angioplastie ayant permis de résorber toute régurgitation de la valve mitrale. Comme on peut le constater aisément, le gradient de l'amplitude du pic PEA1 par rapport à la fréquence cardiaque Fc est beaucoup plus élevé après l'intervention, donc après disparition de la pathologie spécifique. C'est ce gradient et sa modification qui seront utilisés pour évaluer l'état du patient, de la manière que l'on exposera plus bas.

La Figure 2 illustre trois chronogrammes de signaux d'électrogramme EGM et d'accélération endocardiaque EA, relevés expérimentalement sur un animal sain, puis avec occlusion progressive d'une artère coronaire. La réduction, consécutive à la réduction du débit de l'artère coronaire, de l'intervalle Ts séparant les deux composantes EA1 et EA2 est visible de façon tout à fait nette, entre la courbe A (avec réduction du débit coronaire) et B (débit coronaire normal).

Cette observation sera utilisée pour évaluer l'évolution de l'état du patient par rapport à une ischémie croissante, de la manière que l'on décrira par la suite.

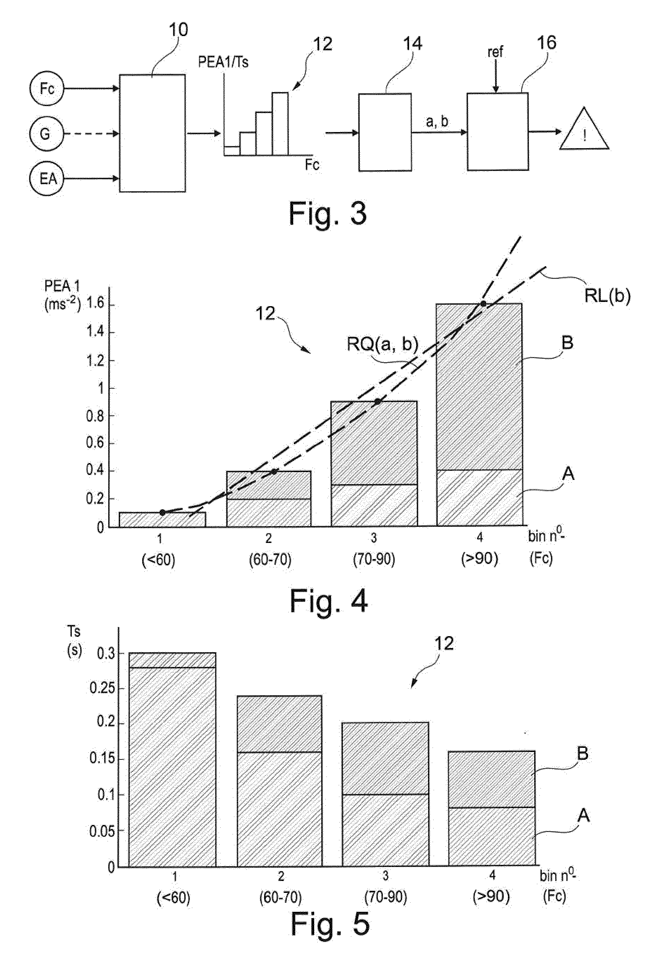

La Figure 3 illustre de façon très schématique les différentes étapes du traitement selon l'invention.

La première étape (bloc 10) consiste à recueillir des signaux de fréquence cardiaque Fc, d'accélération endocardiaque EA, et éventuellement d'activité du patient G (au moyen d'un capteur d'activité physique, typiquement un accéléromètre intégré à l'implant).

Les données sont traitées de manière à extraire des valeurs de paramètres représentatifs tels que PEA1 et/ou Ts, qui seront répartis dans un profil 12 discrétisé (c'est-à-dire d'apparence comparable celle d'un histogramme), en plusieurs classes, en fonction de la fréquence cardiaque Fc. L'étape suivante (bloc 14) consiste à opérer une analyse du profil 12 de manière à en dériver un ou plusieurs indices *a*, *b* ... représentatifs de l'état, plus ou moins altéré, du patient.

Ces indices sont ensuite comparés (bloc 16) à des valeurs de référence *ref* de manière à délivrer si besoin une alerte en cas d'aggravation avérée. La base de comparaison *ref* peut être soit un état antérieur du patient (comparaison relative et intrinsèque, par rapport aux mêmes indices calculés antérieurement pour ce patient), ou bien des valeurs de référence obtenues pour une population connue de patients sains (comparaison absolue, par rapport à des critères donnés déterminés de façon statistique et générale pour une population de patients).

Les Figures 4 et 5 illustrent plus précisément deux exemples de profils 12 constitués à partir des données recueillies par le dispositif.

La plage de fréquences cardiaques possibles du patient est fractionnée en plusieurs sous-plages ou "bins" de fréquences cardiaques, qui peuvent être soit déterminés de la même façon pour tous les patients, soit adaptées en fonction des valeurs propres au patient de fréquence de base et de fréquence maximale en rythme sinusal. Ces différents bins peuvent être de largeur égale ou non.

Dans les exemples illustrés sur les Figures 4 et 5, on a choisi de diviser la plage des fréquences en quatre bins correspondant respectivement à des valeurs de fréquences cardiaques inférieures à 60 bpm, comprises entre 60 et 70 bpm, entre 70 et 90 bpm, et supérieures à 90 bpm.

Le rythme cardiaque courant est mesuré de façon continue, sur la base des intervalles RR. La valeur Fc retenue peut être avantageusement une moyenne pondérée des fréquences cardiaques de cycles précédents, et/ou de valeurs précédemment calculées de Fc.

Les paramètres caractéristiques du signal EA, à savoir PEA1 et/ou Ts dans les exemples illustrés, sont évalués à chaque cycle et cumulés dans l'un des bins du profil 12, en fonction de la valeur Fc correspondante. Ce cumul des paramètres PEA1 et/ou Ts peut prendre la forme d'une valeur moyenne ou médiane, ou être simplement constitué par la somme des valeurs recueillies, le nombre correspondant de cycles concernés étant connu par ailleurs.

Le processus de cumul est suspendu pendant un ou plusieurs cycles en cas de survenue de certains évènements, par exemple la détection d'une extrasystole auriculaire (ESA) ou ventriculaire (ESV) ou d'une arythmie auriculaire ou ventriculaire.

La Figure 4 montre plus précisément un profil du paramètre PEA1 en fonction de la fréquence cardiaque Fc, classé sur quatre bins de fréquence. On a représenté deux exemples de profils, l'un A correspondant à un patient souffrant de régurgitation de la valvule mitrale, et l'autre B obtenu pour un patient sain.

La Figure 5 est homologue de la Figure 4, à la différence que le paramètre évalué est ici la durée Ts séparant les deux pics d'accélération endocardiaque, correspondant à la durée de la systole. On a représenté deux exemples de profils, l'un A correspondant à un patient ischémique,

et l'autre B obtenu pour un patient sain. Comme on l'a exposé plus haut, les patients ischémiques présentent une période systolique plus courte que les patients normaux.

Dans tous les cas, pour un patient donné le dispositif mémorise deux profils, à savoir un profil court terme basé par exemple sur les relevés de la journée précédente ou des deux journées précédentes, et un profil long terme basé par exemple sur le cumul des paramètres relevés sur un mois (en variante, le profil long terme peut être remplacé par un profil de référence obtenu à partir d'une population de patients sains).

L'évaluation de l'état du patient est opérée en comparant le profil court terme et le profil long terme. Cette comparaison peut être réalisée par diverses méthodes connues, avantageusement avec pondération des valeurs de chaque bin de fréquence : par exemple 10 % pour le bin n°1 (< 60 bpm), 20 % pour le bin n°2 (60-70 bpm), 30 % pour le bin n°3 (70 et 90 bpm et 40 % pour le bin n°4 (>90 bpm). L'algorithme calcule la différence entre le profil long terme et le profil court terme pour chaque bin et la multiplie par la pondération associée à ce bin, puis somme les valeurs obtenues pour tous les bins. On obtient ainsi un indice représentatif de la différence entre le profil long terme et le profil court terme.

Une autre manière de calculer un tel indice représentatif consiste à modéliser chacun des profils long terme et court terme par exemple par une régression linéaire RL de la forme :

$$PEA1 = b.F_c + c$$

ou par une régression quadratique RQ de la forme :

$$PEA1 = a.F_c^2 + b.F_c + c$$

où $b$ représente le gradient linéaire du profil et, dans le cas d'une régression quadratique, $a$ indique la courbure du profil.

Un gradient linéaire $b$ élevé et un coefficient quadratique $a$ élevé sont révélateurs d'un état satisfaisant du patient. Par comparaison des deux profils court terme et long terme, on peut évaluer l'évolution de l'état du patient :

- une augmentation des indices $a$ ou $b$ du court terme par rapport au long terme révèle une amélioration de l'état du patient ;

- des indices $a$ et $b$ comparables entre le court terme et le long terme indiquent une stabilité de l'état du patient ; et

- une diminution des indices $a$ ou $b$ du court terme par rapport au long terme révèle une détérioration de l'état du patient.

Dans ce dernier cas, il est possible de générer une alerte, à transmettre via un système de télétransmission à un médecin ou un intervenant extérieur.

Cette alerte peut être générée simplement par comparaison des indices $a$ et/ou $b$ avec un seuil donné, déterminé *a priori* à partir d'une population connue de patients, ou bien par une analyse de tendance de l'écart court terme/long terme sur la durée : par exemple, une alerte peut être générée si la différence mesurée entre le profil court terme et le profil long terme a été négative pendant cinq jours consécutifs. Ces données peuvent être également mémorisées pour être présentées au médecin par télétransmission ou lors d'une interrogation de l'implant lors d'une visite ou à l'admission à l'hôpital.

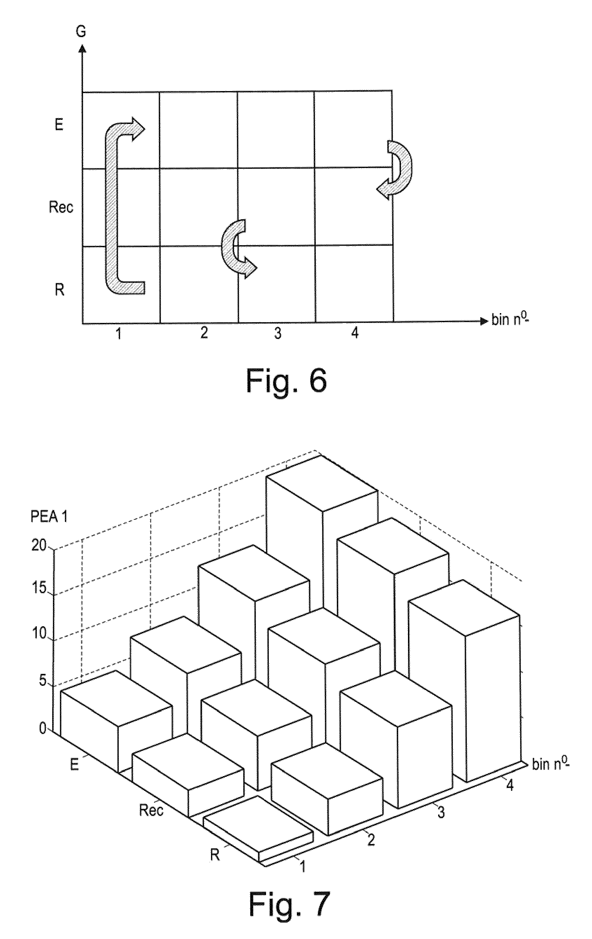

Les Figures 6 et 7 illustrent un perfectionnement consistant à considérer un profil fonction non seulement de la fréquence cardiaque Fc, mais également de l'état d'activité du patient.

Comme illustré Figure 6, on définit par exemple trois état d'activité, en fonction de l'indication donnée par un capteur tel qu'un capteur accélérométrique (capteur G) intégré à l'implant :

- "repos" R : valeur basse du capteur d'activité ;
- "effort" E : valeur haute du capteur d'activité ; et
- "récupération" REC : par exemple pendant une période fixe donnée suivant une période "d'effort", et/ou jusqu'à ce que la fréquence cardiaque du patient redescende à une fréquence de repos spécifiée.

La Figure 6 illustre par exemple l'évolution de l'état du patient, suivant les flèches : en partant d'une situation stable de repos et rythme lent (binl/état R), le patient se met en mouvement (binl/état E) ce qui induit une augmentation de la fréquence cardiaque (jusqu'à bin4/état E). Lorsque le patient met fin à son effort, il passe à l'état de récupération et la fréquence cardiaque redescend progressivement (bin4/état REC, puis bin3/état REC). À la fin du temps de récupération prescrit, l'état devient un état de repos (bin3/état R), puis la fréquence cardiaque redescend progressivement, toujours à l'état de repos, jusqu'à la fréquence de base correspondant à la situation initiale (binl/état R).

On définit, comme illustré Figure 7, un profil tridimensionnel comportant 4 x 3 bins, à savoir 4 bins de fréquence (comme sur la Figure 4) combinés avec 3 bins d'activité (activité/repos/récupération).

L'algorithme analyse ce profil tridimensionnel et son évolution court terme/long terme suivant des techniques comparables à celles exposées plus haut à propos du profil bidimensionnel de la Figure 4, de manière à apprécier l'évolution de la pathologie du patient.

**Revendications**

1. Un dispositif médical actif, notamment un dispositif implantable de stimulation, resynchronisation et/ou défibrillation, ou un appareil à visée diagnostique, comportant :

- des moyens de mesure de la fréquence cardiaque (Fc) ;
- des moyens de recueil d'un signal d'accélération endocardiaque (EA) ;
- des moyens aptes à dériver du signal d'accélération endocardiaque un paramètre hémodynamique représentatif de la contractilité du myocarde ;
- des moyens d'acquisition, aptes à définir au moins des couples de valeurs {fréquence, paramètre} au cours de cycles cardiaques successifs, ou de séries successives de cycles cardiaques ;
- des moyens classificateurs (10), aptes à distribuer lesdits couples de valeurs dans des classes de fréquence prédéterminées d'un profil discrétisé de valeurs {fréquence, paramètre} (12) ; et
- des moyens (14) d'analyse dudit profil, aptes à délivrer un indice (a, b) représentatif d'un état clinique du patient,
**caractérisé en ce que** ledit paramètre hémodynamique représentatif de la contractilité du myocarde est un paramètre temporel représentatif de l'intervalle de temps (Ts) séparant le premier et le second pic d'accélération endocardiaque.

2. Le dispositif de la revendication 1, dans lequel les moyens d'analyse du profil sont des moyens aptes à extraire au moins une donnée (a, b) représentative du profil courant du patient, et à comparer cette donnée représentative courante à une donnée représentative de référence prédéterminée.

3. Le dispositif de la revendication 2, dans lequel ladite donnée représentative de référence est une donnée représentative obtenue à partir d'au moins un profil antérieur du patient.

4. Le dispositif de la revendication 2, dans lequel ladite donnée représentative de référence est une donnée représentative issue de valeurs recueillies auprès d'une population connue de patients sains.

5. Le dispositif de la revendication 2, dans lequel les moyens d'analyse sont en outre aptes, avant comparaison à la donnée représentative de référence, à appliquer une pondération prédéterminée à chaque classe de profil.

6. Le dispositif de la revendication 2, dans lequel les moyens d'analyse sont des moyens aptes à modéliser le profil courant par une régression linéaire (RL) ou quadratique (RQ), les paramètres de cette régression formant lesdites données de profil (a, b).

7. Le dispositif de la revendication 1, dans lequel :

- le dispositif comprend en outre des moyens discriminateurs de phases d'activité du patient (E, R, Rec) ;
- les moyens d'acquisition sont aptes à définir des triplets de valeurs {fréquence, paramètre, activité} au cours desdits cycles cardiaques successifs, ou desdites séries successives de cycles cardiaques ;
- le profil est un profil tridimensionnel discrétisé de valeurs {fréquence, paramètre, activité} ; et
- les moyens classificateurs sont aptes à placer lesdits triplets de valeurs dans des classes de fréquence prédéterminées dudit profil tridimensionnel.

8. Le dispositif de la revendication 7, dans lequel lesdites phases discriminées d'activité du patient comprennent des phases d'effort (E), de repos (R) et de récupération (Rec).

## Patentansprüche

1. Aktive medizinische Vorrichtung, insbesondere eine implantierbare Stimulations-, Resynchronisations- und/oder Defibrillationsvorrichtung, oder ein diagnostisches Gerät, die aufweist:

   - Einrichtungen zur Messung der Herzfrequenz (Fc);
   - Einrichtungen zum Auffangen eines Signals endokardialer Beschleunigung (EA);
   - Einrichtungen, die vom Signal endokardialer Beschleunigung einen für die Kontraktilität des Myokards repräsentativen hämodynamischen Parameter ableiten können;
   - Erfassungseinrichtungen, die mindestens Wertepaare {Frequenz, Parameter} während aufeinanderfolgender Herzzyklen oder aufeinanderfolgender Reihen von Herzzyklen definieren können;
   - Klassifizierungseinrichtungen (10), die die Wertepaare in vorbestimmten Frequenzklassen eines diskretisierten Profils von Werten {Frequenz, Parameter} (12) verteilen können; und
   - Einrichtungen (14) zur Analyse des Profils, die einen für einen klinischen Zustand des Patienten repräsentativen Index (a, b) liefern können, **dadurch gekennzeichnet, dass** der für die Kontraktilität des Myokards repräsentative hämodynamische Parameter ein für das die erste und die zweite endokardiale Beschleunigungsspitze trennende Zeitintervall (Ts) repräsentativer zeitlicher Parameter ist.

2. Vorrichtung nach Anspruch 1, wobei die Einrichtungen zur Analyse des Profils Einrichtungen sind, die mindestens einen für das laufende Profil des Patienten repräsentativen Datenwert (a, b) entnehmen und diesen laufenden repräsentativen Datenwert mit einem vorbestimmten repräsentativen Bezugsdatenwert vergleichen können.

3. Vorrichtung nach Anspruch 2, wobei der repräsentative Bezugsdatenwert ein ausgehend von mindestens einem früheren Profil des Patienten erhaltener repräsentativer Datenwert ist.

4. Vorrichtung nach Anspruch 2, wobei der repräsentative Bezugsdatenwert ein von Werten stammender repräsentativer Datenwert ist, die bei einer bekannten Population gesunder Patienten gesammelt wurden.

5. Vorrichtung nach Anspruch 2, wobei die Analyseeinrichtungen außerdem vor dem Vergleich mit dem repräsentativen Bezugsdatenwert eine vorbestimmte Gewichtung an jede Profilklasse anwenden können.

6. Vorrichtung nach Anspruch 2, wobei die Analyseeinrichtungen Einrichtungen sind, die das laufende Profil durch eine lineare (RL) oder quadratische (RQ) Regression modellisieren können, wobei die Parameter dieser Regression die Profildaten (a, b) bilden.

7. Vorrichtung nach Anspruch 1, wobei:

   - die Vorrichtung außerdem Unterscheidungseinrichtungen von Aktivitätsphasen des Patienten (E, R, Rec) enthält;
   - die Erfassungseinrichtungen Werte-Triplets {Frequenz, Parameter, Aktivität} während der aufeinanderfolgenden Herzzyklen oder der aufeinanderfolgenden Reihen von Herzzyklen definieren können;
   - das Profil ein diskretisiertes dreidimensionales Profil von Werten {Frequenz, Parameter, Aktivität} ist; und
   - die Klassifizierungseinrichtungen die Werte-Triplets in vorbestimmten Frequenzklassen des dreidimensionalen Profils einordnen können.

8. Vorrichtung nach Anspruch 7, wobei die unterschiedenen Aktivitätsphasen des Patienten Phasen der Anstrengung (E), der Ruhe (R) und der Erholung (Rec) enthalten.

## Claims

1. An active medical device, in particular an implantable stimulation, resynchronisation and/or defibrillation device, or a device for diagnosis purpose, including:

   - means for measuring the heart rate (Fc);
   - means for collecting an endocardial acceleration (EA) signal;
   - means adapted to derive from the endocardial acceleration signal an hemodynamic parameter representative of the myocardial contractility;

- acquisition means, adapted to define at least couples of values {rate, parameter} during successive cardiac cycles, or successive series of cardiac cycles;
- classifying means (10), adapted to distribute said couples of values into predetermined rate classes of a discrete profile of values {rate, parameter} (12); and
- means (14) for analysing said profile, adapted to deliver an index (a, b) representative of a clinical state of the patient,
**characterized in that** said hemodynamic parameter representative of the myocardial contractility is a time parameter representative of the time interval (Ts) separating the first and the second peaks of endocardial acceleration.

2. The device of claim 1, wherein the profile analysing means are means adapted to extract at least one datum (a, b) representative of the current patient's profile, and to compare this current representative datum with a predetermined reference representative datum.

3. The device of claim 2, wherein said reference representative datum is a representative datum obtained from at least one prior profile of the patient.

4. The device of claim 2, wherein said reference representative datum is a representative datum issued from values collected with a known population of healthy patients.

5. The device of claim 2, wherein the analysis means are further adapted, before comparison with the reference representative datum, to apply a predetermined ponderation to each class of profile.

6. The device of claim 2, wherein the analysis means are means adapted to model the current profile by a linear (RL) or quadratic (RQ) regression, the parameters of this regression forming said profile data (a, b).

7. The device of claim 1, wherein:

- the device further comprises means for discriminating patient's activity phases (E, R, Rec);
- the acquisition means are adapted to define triplets of values {rate, parameter, activity} during said successive cardiac cycles, or said successive series of cardiac cycles;
- the profile is a discrete three-dimensional profile of values {rate, parameter, activity}; and
- the classifying means are adapted to place said triplets of values into predetermined rate classes of said three-dimensional profile.

8. The device of claim 7, wherein said discriminated patient's activity phases comprise phases of effort (E), of rest (R) and of recovery (Rec).

Fig. 1

Fig. 2

Fig. 3

Fig. 4

Fig. 5

Fig. 6

Fig. 7

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1867360 A2
- US 2007043299 A1
- EP 0515319 A1
- EP 2092885 A1